# EUROPEAN PATENT SPECIFICATION

(11) **EP 3 667 802 B1**
(45) Date of publication and mention of the grant of the patent: **08.01.2025**
(21) Application number: 18843138.1
(22) Date of filing: 06.08.2018
(51) Int. Cl.: H01M 10/0567, H01M 10/0525, H01G 11/06, H01G 11/64, C07D 333/48, C07D 335/02

(54) **ADDITIVE FOR NON-AQUEOUS ELECTROLYTE, NON-AQUEOUS ELECTROLYTE, AND POWER STORAGE DEVICE**
ADDITIV FÜR NICHTWÄSSRIGEN ELEKTROLYTEN, NICHTWÄSSRIGER ELEKTROLYT UND STROMSPEICHERVORRICHTUNG
ADDITIF POUR UN ÉLECTROLYTE NON-AQUEUX, ÉLECTROLYTE NON-AQUEUX ET DISPOSITIF DE STOCKAGE D'ÉNERGIE

(30) Priority: 08.08.2017 JP 2017153451
(43) Date of publication of application: 17.06.2020
(73) Proprietor: Sumitomo Seika Chemicals Co., Ltd., Kako-gun, Hyogo 675-0145 (JP)
(72) Inventor: FUJIMOTO, Shohei, Kako-gun Hyogo 675-0145 (JP); KONO, Yuki, Kako-gun Hyogo 675-0145 (JP); FUJITA, Koji, Kako-gun Hyogo 675-0145 (JP)
(74) Representative: Nederlandsch Octrooibureau
(86) International application number: PCT/JP2018/029421
(87) International publication number: WO 2019/031452

(56) References cited:
- EP-A1- 1 864 976
- EP-A1- 2 833 381
- WO-A1-2013/026930
- WO-A1-2015/087963
- WO-A1-2017/043576
- CN-A- 102 496 467
- JP-A- 2003 132 944
- JP-A- 2012 056 925
- JP-A- 2012 106 987
- JP-A- 2014 528 639
- JP-A- 2016 192 362
- H.E. FAITH ET AL: "TETRAHYDROTHIOPHENE 1,1-DIOXIDE DERIVATIVES", JOURNAL OF ORGANIC CHEMISTRY, vol. 27, 1 August 1962 (1962-08-01), pages 2889 - 2897, XP000602006, ISSN: 0022-3263, DOI: 10.1021/JO01055A040

## Description

### Technical Field

The present invention relates to an additive for nonaqueous electrolyte solutions, a nonaqueous electrolyte solution, and an electricity storage device.

### Background Art

In recent years, along with an increase in attention to solving environmental problems and establishing a sustainable recycling-based society, nonaqueous electrolyte solution secondary batteries typified by lithium ion batteries have been extensively studied. The lithium ion batteries have been used as the power sources for laptops, mobile phones, and the like from the viewpoint that they have high working voltages and energy densities. The lithium ion batteries are promising to establish a higher battery capacity from the viewpoint that they have higher energy densities than lead batteries and nickel-cadmium batteries.

However, the lithium ion batteries have a problem in that the capacity of the batteries is decreased over time in charging and discharging cycles. It is considered that factors contributing a decrease in the capacity are, for example, decomposition of an electrolyte solution due to an electrode reaction caused by long-term charge-discharge cycles, a decrease in impregnation of an electrolyte into an electrode active material layer, and a decrease in the efficiency of lithium ion intercalation.

As a method for suppressing such a decrease in a battery capacity from charging and discharging cycles, a method in which various additives are added to an electrolyte solution has been examined. The additives are generally decomposed during an initial charging and discharging cycle to form a film called a solid electrolyte interface (SEI) on the surface of an electrode. Since the SEI is formed during the initial charging and discharging cycle, consumption of the electricity for decomposition of the electrolyte solution in the subsequent charge and discharge and the lithium ions can transfer between the electrodes through the SEI. That is, it is considered that the formation of the SEI plays a major role in suppressing the deterioration of a secondary battery in a case of repeating charging and discharging cycles and in improving battery characteristics, storage characteristics, load characteristics, and the like.

As an additive for an electrolyte solution, for example, Patent Literatures 1 to 3 each disclose a cyclic monosulfonic acid ester, Patent Literature 4 discloses a sulfur-containing aromatic compound, and Patent Literatures 5 to 8 each disclose a disulfonic acid ester. In addition, Patent Literatures 9 to 13 each disclose an electrolyte solution containing a cyclic carbonic acid ester or a cyclic sulfone.

Non-Patent Literature 2 and Patent Literatures 14 and 15 describe the preparation or the use of cyano-substituted cyclic sulfones for pharmaceutical or pesticidal applications.

Patent Literature 16 discloses the use of α-cyanosulfolane as additive for a non-aqueous electrolytic solution in a supercapacitor.

Patent Literatures 17 to 21 all refer to non-aqueous electrolytic solutions for electrochemical devices, comprising a lithium salt dissolved in a non-aqueous organic solvent, in admixture with a substituted sulfolane compound as co-solvent or additive.

### Citation List

### Patent Literature

[Patent Literature 1] Japanese Unexamined Patent Publication No. S63-102173
[Patent Literature 2] Japanese Unexamined Patent Publication No. 2000-003724
[Patent Literature 3] Japanese Unexamined Patent Publication No. H11-339850
[Patent Literature 4] Japanese Unexamined Patent Publication No. H05-258753
[Patent Literature 5] Japanese Unexamined Patent Publication No. 2009-038018
[Patent Literature 6] Japanese Unexamined Patent Publication No. 2005-203341
[Patent Literature 7] Japanese Unexamined Patent Publication No. 2004-281325
[Patent Literature 8] Japanese Unexamined Patent Publication No. 2005-228631
[Patent Literature 9] Japanese Unexamined Patent Publication No. H04-87156
[Patent Literature 10] Japanese Unexamined Patent Publication No. H08-45545
[Patent Literature 11] Japanese Unexamined Patent Publication No. 2001-6729
[Patent Literature 12] Japanese Unexamined Patent Publication No. H05-074486
[Patent Literature 13] PCT International Publication No. WO2017/043576
[Patent Literature 14] : WO 2013/026930 A1
[Patent Literature 15] : EP 1 864 976 A1
[Patent Literature 16] : CN 102 496 467 A
[Patent Literature 17] : EP 2 833 381 A1
[Patent Literature 18] : JP 2012 056925 A
[Patent Literature 19] : JP 2003 132944 A
[Patent Literature 20] : JP 2012 106987 A
[Patent Literature 21] : JP 2014 528639 A

### Non Patent Literature

[Non-Patent Literature 1] Geun-Chang, Hyung-Jin kim, Seung-ll Yu, Song-Hui Jun, Jong-Wook Choi, Myung-Hwan Kim. Journal of The Electrochemical Society, 147, 12, 4391 (2000)
[Non-Patent Literature 2] : H.E. Faith et al., Journal of Organic Chemistry, vol. 27, 1 August 1962 (1962-08-01), pages 2889-2897

### Summary of Invention

### Technical Problem

It is considered that a compound having a low lowest unoccupied molecular orbital (LUMO) energy is an excellent electron receptor and can form a stable SEI on the surface of an electrode in a nonaqueous electrolyte solution secondary battery and the like (for example, Non-Patent Literature 1).

Some of the additives in the related art as disclosed in Patent Literatures 1 to 8 exhibit a low LUMO energy, but there is room for an improvement in terms of chemical stability, such as improvement of easy deterioration caused by an influence of moisture and temperature. For example, a disulfonic acid ester exhibits a low LUMO energy, but has low stability against moisture and is easily deteriorated, for which there is a demand for strict management of a moisture content and a temperature in a case of storage long-term storage. In addition, in general, from the viewpoint that a heat-resistant temperature of about 60°C for a lithium ion battery and a heat-resistant temperature of about 80°C for a lithium ion capacitor are demanded, it has been one of important objects to improve the high-temperature stability of an additive for nonaqueous electrolyte solutions used in an electricity storage device.

In a case of an electrolyte solution containing the additive in the related art, the battery characteristics of an electricity storage device are easily deteriorated during a use of the electricity storage device over a long period of time while repeating charging and discharging cycles. As a result, there has been a demand for a further improvement in terms of cycle characteristics.

The electrolyte solutions described in Patent Literatures 9 to 11 can suppress an irreversible decrease in a capacity to some extent by an SEI generated on the surface of a negative electrode by electrochemical reduction decomposition. However, the SEI formed by these additives was superior in performance to protect the electrodes, but was not sufficient in terms of strength to withstand a long-term use. As a result, there has been a problem in that the surface of the negative electrode is exposed by decomposition of the SEI or generation of cracks in the SEI during a use of the electricity storage device, and the decomposition of the electrolyte solution occurs, whereby the battery characteristics are deteriorated. An electrolyte solution using the ethylene carbonate-based compound described in Patent Literature 12 as an additive had a problem in that upon decomposition of ethylene carbonate on the electrode, a gas including carbon dioxide is generated, leading to deterioration in the battery performance. Gas generation is particularly remarkable in a case of repeating the charging and discharging cycles at a high temperature or over a long period of time.

As such, with regard to the additive for nonaqueous electrolyte solutions, there has been room for a further improvement in terms of storage stability, cycle characteristics of maintaining the performance in a case of repeating charging and discharging cycles, or suppression of gas generation.

Therefore, an object of the present invention is to provide an additive for nonaqueous electrolyte solutions which has high storage stability and enables improvement of cycle characteristics and suppression of gas generation for an electricity storage device.

### Solution to Problem

In one aspect of the present invention, provided is the use of an additive in nonaqueous electrolyte solutions of a lithium-ion battery, said additive comprising a compound represented by Formula (1).

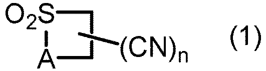

In Formula (1), A represents a divalent hydrocarbon group having 1 to 3 carbon atoms, which may be substituted with a halogen atom. n represents an integer of 1 to 5. In the present specification, an expression, "which may be substituted with a halogen atom", means that a hydrogen atom included in each group may be substituted with a halogen atom.

In another aspect of the present invention, provided is a lithium-ion battery comprising a nonaqueous electrolyte solution containing the additive defined here above a nonaqueous solvent, and an electrolyte, and further comprising a positive electrode, and a negative electrode.

### Advantageous Effects of Invention

According to the present invention, it is possible to provide an additive for nonaqueous electrolyte solutions, which has high storage stability and enables improvement of cycle characteristics and suppression of gas generation for an electricity storage device. In addition, the additive for nonaqueous electrolyte solutions of the present invention can form a stable solid electrolyte interface (SEI) on the surface of an electrode to improve battery characteristics such as cycle characteristics, a charging and discharging capacity, and internal resistance in a case where the additive for nonaqueous electrolyte solutions is used in an electricity storage device such as a nonaqueous electrolyte solution secondary battery and an electric double layer capacitor.

### Brief Description of Drawings

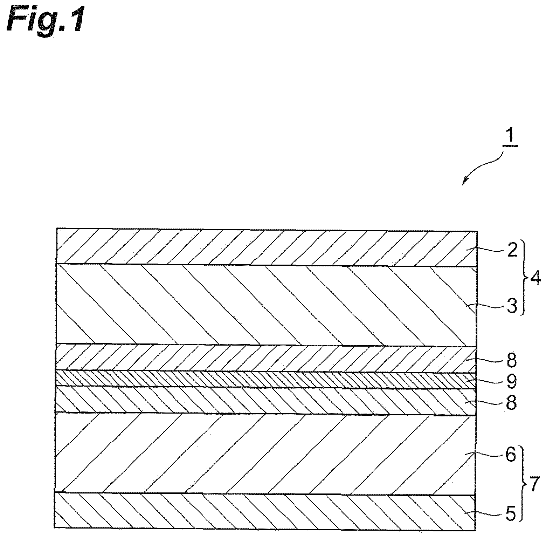

Fig. 1 is a cross-sectional view showing an embodiment of a nonaqueous electrolyte solution secondary battery as an example of an electricity storage device.

### Description of Embodiments

Hereinafter, some embodiments of the present invention will be described in detail. It should be noted that the present invention is not limited to the following embodiments.

An additive for nonaqueous electrolyte solutions according to one embodiment includes one kind or two or more kinds of compounds represented by Formula (1).

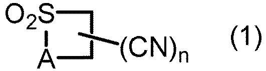

In Formula (1), A represents a divalent hydrocarbon group having 1 to 3 carbon atoms, which may be substituted with a halogen atom. n represents an integer of 1 to 5. That is, the compound of Formula (1) has a sulfonyl group (-S(=O)₂-), a cyclic group with a 4- to 6-membered ring, including an ethylene group (-CH₂CH₂-) and A, and n pieces of nitrile groups bonded to the cyclic group. n may be an integer of 1 to 4, or 1 to 3. The nitrile group only needs to be bonded to any of carbon atoms constituting the hydrocarbon group of A or the ethylene group in the cyclic group. Two nitrile groups may be bonded to one carbon atom.

The compound of Formula (1) is susceptible to electrochemical reduction, since it exhibits a low LUMO energy. As a result, a nonaqueous electrolyte solution containing the compound as an additive for nonaqueous electrolyte solutions can form a stable SEI on the surface of an electrode to improve battery characteristics such as cycle characteristics, a charging and discharging capacity, and internal resistance in a case where the compound is used in an electricity storage device such as a nonaqueous electrolyte solution secondary battery. In addition, since the compound of Formula (1) is stable against a change in moisture and temperature, an additive for nonaqueous electrolyte solutions and a nonaqueous electrolyte solution, each including the compound, can be stored at room temperature for a long period of time.

The lowest unoccupied molecular orbital (LUMO) energy of the compound represented by Formula (1) may be -3.0 eV or more and may be 0.0 eV or less. In a case where the LUMO energy is -3.0 eV or more, there is no case where the compound is not excessively decomposed, and as a result, it is difficult to form an SEI exhibiting high resistance in some cases. In a case where the LUMO energy is 0.0 eV or less, it is possible to more easily form a more stable SEI on the surface of a negative electrode. From the same viewpoint, the LUMO energy may be -2.0 eV or more and may be -0.1 eV or less. Those skilled in the related art can discover a compound exhibiting a LUMO energy within these numerical value ranges as long as it falls within the scope of the compound defined by Formula (1) without undue trials and errors.

In the present specification, the "lowest unoccupied molecular orbital (LUMO) energy" is a value calculated by the combination of PM3 which is a semi-empirical molecular orbital calculation method with a B3LYP method which is a density-functional theory calculation method. Specifically, the LUMO energy can be calculated using Gaussian 03 (Revision B. 03, software manufactured by Gaussian, Inc., USA).

A in Formula (1) may be an alkylene group having 1 to 3 carbon atoms, which is substituted with a halogen atom. The number of carbon atoms contained in the hydrocarbon group (particularly alkylene group) included in A may be 1 to 3, or 2 or 3. Specific examples of A include -CH₂-, -CH₂CH₂-, -CH₂CH₂CH₂-, -CFHCH₂-, -CF₂CH₂-, and -CF₂CH₂CH₂-.

Examples of the halogen atom include an iodine atom, a bromine atom, and a fluorine atom. From the viewpoint that the battery resistance is further decreased, a fluorine atom can be selected as the halogen atom.

The compound of Formula (1) may be a compound represented by Formula (1a). The compound represented by Formula (1a) particularly tends to exhibit a low LUMO energy and exert a more excellent ion conductivity. A in Formula (1a) has the same definition as A in Formula (1).

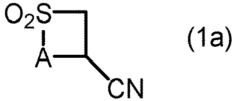

From the viewpoint that the battery resistance is more easily lowered, the compound of Formula (1) may be a compound represented by Formula (5). n in Formula (5) represents an integer of 1 to 4, and may be an integer of 1 to 3. In Formula (5), one nitrile group may be bonded to a carbon atom at the 3-position based on the sulfonyl group at the 1-position, as in Formula (1a).

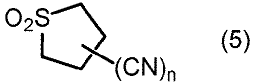

The additive for nonaqueous electrolyte solutions may include at least one compound of compounds represented by Formulae (5a), (5b), (5c), (5d), (5e), (6a), (6b), (6c), and (6d) as the compound represented by Formula (1). Among those, in particular, the compound of Formula (5a) may be selected.

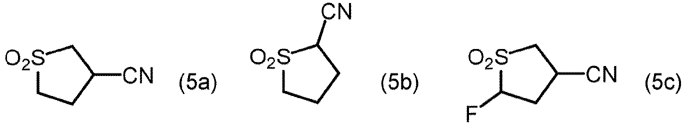

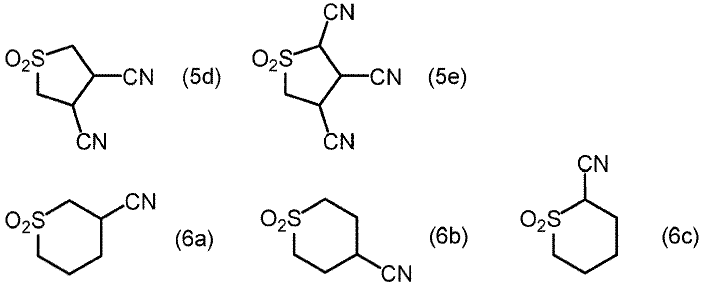

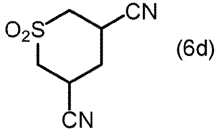

The compound represented by Formula (1) can be synthesized by using available raw materials and combining common reactions. For example, the compound of Formula (5a) which is one of specific examples can be synthesized in accordance with a method of reacting a metal cyanide such as potassium cyanide or a cyanating agent such as acetone cyanohydrin with sulfolene.

Specific examples of a case of producing the compound of Formula (5a) are set forth below. First, 3-sulfolene and triethylamine are dissolved in an organic solvent such as isopropyl alcohol, acetone cyanohydrin is added dropwise to the obtained reaction solution, and the reaction solution is stirred at room temperature for 2 hours. Thereafter, water is added to the obtained reaction product to perform liquid separation, and the oily layer is concentrated, whereby a desired compound can be obtained.

The compound in which A in Formula (1) is an alkylene group having 1 to 3 carbon atoms, substituted with a halogen atom, can be produced by reacting a compound obtained by the production method with a fluorinating agent such as N-fluoro-N'-(chloromethyl)-triethylenediaminebis(tetrafluoroborate).

An additive for nonaqueous electrolyte solutions according to one embodiment may include other general components such as a compound contributing to formation of an SEI, in addition to the compound of Formula (1). Alternatively, only the compound of Formula (1) may be used as the additive for nonaqueous electrolyte solutions. Examples of such other general components include vinylene carbonate (VC), fluoroethylene carbonate (FEC), 1,3-propane sultone (PS), a negative electrode protecting agent, a positive electrode protecting agent, a flame retardant, and an anti-overcharging agent.

A nonaqueous electrolyte solution according to one embodiment contains the additive for nonaqueous electrolyte solutions of the above-mentioned embodiment, a nonaqueous solvent, and an electrolyte. The content of the additive (or the compound of Formula (1)) for nonaqueous electrolyte solutions in the nonaqueous electrolyte solution may be 0.005% by mass or more and may be 10% by mass or less, with respect to the total mass of the nonaqueous electrolyte solution. In a case where the content is 0.005% by mass or more, a stable SEI is likely to be sufficiently formed by an electrochemical reaction on the surface of the electrode. In a case where the content is 10% by mass or less, the additive for nonaqueous electrolyte solutions can be easily dissolved in a nonaqueous solvent. Further, it is possible to easily secure the mobility of the ions by not excessively increasing the content of the additive for nonaqueous electrolyte solutions to suppress an increase in the viscosity of the nonaqueous electrolyte solution. In a case where the mobility of the ions is not sufficiently secured, it is impossible to sufficiently secure an electrical conductivity and the like of the nonaqueous electrolyte solution, which may cause a problem in charge-discharge characteristics of the electricity storage device. From the same viewpoint, the content of the additive (or the compound of Formula (1)) for nonaqueous electrolyte solutions may be 0.01% by mass or more, may be 0.1% by mass or more, and may be 0.5% by mass or more. From the same viewpoint, the content of the additive (or the compound of Formula (1)) for nonaqueous electrolyte solutions may be 5% by mass or less and may be 2.0% by mass or less.

The nonaqueous electrolyte solution may include two or more kinds of compounds that form an SEI. In this case, a total content of the compounds may be 0.005% by mass or more and may be 10% by mass or less, with respect to the total mass of the nonaqueous electrolyte solution. Other examples of the compounds that form an SEI include VC, FEC, and PS.

From the viewpoints that for example, the viscosity of a nonaqueous electrolyte solution thus obtained is suppressed to a lower value, an aprotic solvent can be selected as the nonaqueous solvent. The aprotic solvent may be at least one selected from the group consisting of a cyclic carbonate, a chained carbonate, an aliphatic carboxylic acid ester, a lactone, a lactam, a cyclic ether, a chained ether, a sulfone, a nitrile, and a halogen derivative thereof. Among those, as the aprotic solvent, the cyclic carbonate or the chained carbonate can be selected, and a combination of the cyclic carbonate and the chained carbonate can also be selected.

Examples of the cyclic carbonate include ethylene carbonate, propylene carbonate, and butylene carbonate. Examples of the chained carbonate include dimethyl carbonate, diethyl carbonate, and ethyl methyl carbonate. Examples of the aliphatic carboxylic acid ester include methyl acetate, ethyl acetate, methyl propionate, ethyl propionate, methyl butyrate, methyl isobutyrate, and methyl trimethylacetate. Examples of the lactone include γ-butyrolactone. Examples of the lactam include ε-caprolactam and N-methylpyrrolidone. Examples of the cyclic ether include tetrahydrofuran, 2-methyltetrahydrofuran, tetrahydropyran, and 1,3-dioxolane. Examples of the chained ether include 1,2-diethoxyethane and ethoxymethoxyethane. Examples of the sulfone include sulfolane. Examples of the nitrile include acetonitrile. Examples of the halogen derivative include 4-fluoro-1,3-dioxolan-2-one, 4-chloro-1,3-dioxolan-2-one, and 4,5-difluoro-1,3-dioxolan-2-one. These nonaqueous solvents may be used singly or in combination of two or more kinds thereof. These nonaqueous solvents are particularly suitable for, for example, a nonaqueous electrolyte solution secondary battery such as a lithium ion battery and an electric double layer capacitor such as a lithium ion capacitor.

The electrolyte constituting the nonaqueous electrolyte solution may be a lithium salt which serves as an ion source of lithium ions. Above all, the electrolyte may be at least one selected from the group consisting of LiAlCl₄, LiBF₄, LiPF₆, LiClO₄, LiAₛF₆, and LiSbF₆. As the electrolyte, LiBF₄, LiPF₆ or a combination thereof may be selected from the viewpoints that they can increase the ion conductivity of the electrolyte solution due to a high degree of dissociation and have an action of suppressing deterioration of the performance of an electricity storage device by a long-term use due to their oxidation-reduction resistance characteristics, for example. These electrolytes may be used singly or in combination of two or more kinds thereof. LiBF₄ and LiPF₆ may be combined with one or more kinds of each of a cyclic carbonate and a chained carbonate as the nonaqueous solvent. In particular, LiBF₄, LiPF₆, or both thereof may be combined with ethylene carbonate and diethyl carbonate as the nonaqueous solvent.

The concentration of the electrolyte in the nonaqueous electrolyte solution may be 0.1 mol/L or more and may be 2.0 mol/L or less. With regard to the concentration of the electrolyte in the nonaqueous electrolyte solution, in a case where the concentration of the electrolyte is 0.1 mol/L or more, the electrical conductivity and the like of the nonaqueous electrolyte solution are likely to be sufficiently secured. As a result, stable discharge characteristics and charge characteristics are easily obtained for the electricity storage device. In a case where the concentration of the electrolyte is 2.0 mol/L or less, in particular, the ion mobility can be easily sufficiently secured by suppressing an increase in the viscosity of the nonaqueous electrolyte solution. In a case where the mobility of ions is not sufficiently secured, the electrical conductivity and the like of the electrolyte solution cannot be sufficiently secured and a problem in the charging and discharging characteristics and the like of the electricity storage device can potentially arise. From the same viewpoint, the concentration of the electrolyte may be 0.5 mol/L or more and may be 1.5 mol/L or less.

The electricity storage device according to the present embodiment is mainly constituted with the nonaqueous electrolyte solution, a positive electrode, and a negative electrode. Specific examples of the electricity storage device include a nonaqueous electrolyte solution secondary battery (a lithium ion battery and the like) and an electric double layer capacitor (a lithium ion capacitor and the like). The nonaqueous electrolyte solution according to the present embodiment is particularly effective in applications of a lithium ion battery and a lithium ion capacitor.

Fig. 1 is a cross-sectional view schematically showing one embodiment of a nonaqueous electrolyte solution secondary battery as an example of an electricity storage device. A nonaqueous electrolyte solution secondary battery 1 shown in Fig. 1 comprises a positive electrode plate 4 (positive electrode), a negative electrode plate 7 (negative electrode) facing the positive electrode plate 4, a nonaqueous electrolyte solution 8 disposed between the positive electrode plate 4 and the negative electrode plate 7, and a separator 9 provided in the nonaqueous electrolyte solution 8. The positive electrode plate 4 has a positive electrode collector 2 and a positive electrode active material layer 3 provided on a surface of the side of the nonaqueous electrolyte solution 8 of the positive electrode collector 2. The negative electrode plate 7 has a negative electrode collector 5 and a negative electrode active material layer 6 provided on a surface of the side of the nonaqueous electrolyte solution 8 of the negative electrode collector 5. The nonaqueous electrolyte solution 8 can be the nonaqueous electrolyte solution according to the above-mentioned embodiment.

The positive electrode collector 2 and the negative electrode collector 5 may be, for example, a metal foil formed of a metal such as aluminum, copper, nickel, and stainless steel.

The positive electrode active material layer 3 includes a positive electrode active material. The positive electrode active material may be a lithium-containing composite oxide. Examples of the lithium-containing composite oxide include compounds represented by LiMn₂O₄, Li₂FeSiO₄, LiFePO₄, and LiNiₓCo_{y}M_{z}O₂ (x, y, and z are each an integer or fractions of 0 to 3, and M represents Fe, Mn, or Si). The compound represented by LiNiₓCo_{y}M_{z}O₂ may be, for example, LiCoO₂ or LiNi_{1/3}Co_{1/3}Mn_{1/3}O₂.

The negative electrode active material layer 6 includes a negative electrode active material. The negative electrode active material may be, for example, a material capable of absorbing and releasing lithium. Specific examples of such a material include carbon materials such as graphite and amorphous carbon, and oxide materials such as indium oxide, silicon oxide, tin oxide, zinc oxide, and lithium oxide. The negative electrode active material may be a lithium metal, or a metal material capable of forming an alloy together with lithium. Specific examples of the metal capable of forming an alloy together with lithium include Cu, Sn, Si, Co, Mn, Fe, Sb, and Ag. A binary or ternary alloy including any of these metals and lithium can also be used as the negative electrode active material. These negative electrode active materials may be used singly or in combination of two or more kinds thereof.

The separator 9 may be a porous film formed of polyethylene, polypropylene, a fluorine resin, or the like, for example.

### EXAMPLES

Hereinafter, the present invention will be described in more detail with reference to Examples. However, the present invention is not limited to these Examples.

### 1. Preparation of Nonaqueous Electrolyte Solution

### Example 1

Ethylene carbonate (EC) and diethyl carbonate (DEC) were mixed at a volume ratio of EC : DEC=30 : 70 to obtain a nonaqueous mixed solvent. LiPF₆ as an electrolyte was dissolved in the obtained nonaqueous mixed solvent such that the solution reached a concentration of 1.0 mol/L. A compound (5a) shown in Table 1 as the additive for nonaqueous electrolyte solutions was added thereto such that a content ratio of the compound (5a) with respect to the total mass of the solution reached 0.5% by mass, thereby preparing a nonaqueous electrolyte solution.

### Example 2

A nonaqueous electrolyte solution was prepared in the same manner as in Example 1, except that the content ratio of the compound (5a) was changed to 1.0% by mass.

### Example 3

A nonaqueous electrolyte solution was prepared in the same manner as in Example 2, except that a compound (5b) shown in Table 1 was used instead of the compound (5a).

### Example 4

A nonaqueous electrolyte solution was prepared in the same manner as in Example 2, except that a compound (5c) shown in Table 1 was used instead of the compound (5a).

### Example 5

A nonaqueous electrolyte solution was prepared in the same manner as in Example 2, except that a compound (5d) shown in Table 1 was used instead of the compound (5a).

### Example 6

A nonaqueous electrolyte solution was prepared in the same manner as in Example 2, except that a compound (6a) shown in Table 1 was used instead of the compound (5a).

### Example 7

A nonaqueous electrolyte solution was prepared in the same manner as in Example 2, except that a compound (6b) shown in Table 1 was used instead of the compound (5a).

### Example 8

A nonaqueous electrolyte solution was prepared in the same manner as in Example 2, except that a compound (6c) shown in Table 1 was used instead of the compound (5a).

### Comparative Example 1

A nonaqueous electrolyte solution was prepared in the same manner as in Example 1, except that the compound (5a) was not added.

### Comparative Example 2

A nonaqueous electrolyte solution was prepared in the same manner as in Example 2, except that 1,3-propanesultone (PS) [manufactured by Kishida Chemical Co., Ltd.] was used instead of the compound (5a).

### Comparative Example 3

A nonaqueous electrolyte solution was prepared in the same manner as in Example 2, except that vinylene carbonate (VC) [manufactured by Kishida Chemical Co., Ltd.] was used instead of the compound (5a).

### Comparative Example 4

A nonaqueous electrolyte solution was prepared in the same manner as in Comparative Example 3, except that the content ratio of VC was set to 2.0% by mass.

### Comparative Example 5

A nonaqueous electrolyte solution was prepared in the same manner as in Example 2, except that fluoroethylene carbonate (FEC) [manufactured by Kishida Chemical Co., Ltd.] was used instead of the compound (5a).

### Comparative Example 6

A nonaqueous electrolyte solution was prepared in the same manner as in Comparative Example 5, except that the content ratio of FEC was set to 2.0% by mass.

### Comparative Example 7

A nonaqueous electrolyte solution was prepared in the same manner as in Example 2, except that sulfolane (manufactured by Sumitomo Seika Chemicals Co., Ltd.) was used instead of the compound (5a).

### Comparative Example 8

### Synthesis of 1,1-Dioxotetrahydro-1-thiophen-3-yl Methanesulfonate Ester (Compound A)

27.2 g (0.20 mol) of hydroxysulfolane and 34.5 g (0.44 mol) of pyridine were put into a 500-mL four-necked flask comprising a stirrer, a condenser, a thermometer, and a dropping funnel to prepare a mixed solution. 25.1 g (0.44 mol) of methanesulfonyl chloride was added dropwise to the mixed solution over 20 minutes while maintaining the temperature of the mixed solution to 0°C. Subsequently, the mixed solution was stirred for 3 hours while maintaining the same temperature.

Next, the mixed solution was filtered and 50.0 g of acetonitrile was added to the filtrate. 200.0 g of water was further added dropwise thereto to precipitate a crystal. The crystal was collected by filtration and the obtained crystal was dried to obtain a compound A (35.5 g, 0.17 mol). The yield of the compound A was 82.9% with respect to hydroxysulfolane.

### Preparation of Nonaqueous Electrolyte Solution

A nonaqueous electrolyte solution was prepared in the same manner as in Example 2, except that the compound A was used instead of the compound (5a).

### Comparative Example 9

### Synthesis of 1,1-Dioxotetrahydro-1-thiophen-3-yl benzenesulfonate Ester (Compound B)

A compound B (44.2 g, 0.16 mol) was obtained in the same manner as in Comparative Example 8, except that 38.8 g (0.44 mol)benzenesulfonyl chloride was used instead of methanesulfonyl chloride.

### Preparation of Nonaqueous Electrolyte Solution

A nonaqueous electrolyte solution was prepared in the same manner as in Example 2, except that the compound B was used instead of the compound (5a).

### 2. Evaluation of Compound

### LUMO Energy

The lowest unoccupied molecular orbital (LUMO) energy of the compound used in Examples was determined by semi-empirical molecular orbital calculation using a Gaussian03 software. The calculated LUMO energy is shown in Table 1.

**Table 1**

| Compound | Structural formula | LUMO energy (eV) | Compound | Structural formula | LUMO energy (eV) |
|---|---|---|---|---|---|
| 5a | 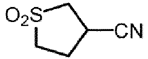 | -0.49 | 6a | 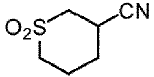 | -0.46 |
| 5b | 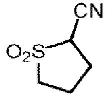 | -0.55 | 6b | 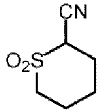 | -0.51 |
| 5c | 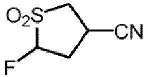 | -0.69 | 6c | 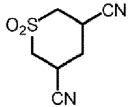 | -0.54 |
| 5d | 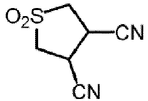 | -0.62 | | | |

### Stability

The compounds used in Examples and the FEC used in Comparative Examples 5 and 6 were subjected to a storage test for 90 days under constant temperature/constant humidity environments of a temperature of 40±2°C and a humidity of 75±5%. The ¹H-nuclear magnetic resonance (¹H-NMR) spectrum of each additive for nonaqueous electrolyte solutions before and after the storage test was measured, and the stability of each compound was evaluated in accordance with the following standard. Table 2 shows the evaluation results of the stability.
A: There was no change in the peaks of the ¹H-NMR spectrum before and after the storage test.
B: A slight change in the peaks of the ¹H-NMR spectrum before and after the storage test was found.
C: An obvious change in the peaks of the ¹H-NMR spectrum before and after the storage test was found.

**Table 2**

| Additive | Stability |
|---|---|
| Compound 5a | A |
| Compound 5b | A |
| Compound 5c | A |
| Compound 5d | A |
| Compound 6a | A |
| Compound 6b | A |
| Compound 6c | A |
| FEC | C |

As shown in Table 2, the FEC used in Comparative Examples 5 and 6 was considered to be partially hydrolyzed and had deteriorated stability. On the other hand, the compounds (5a) to (5d), and (6a) to (6c) used in Examples showed almost no change in the peaks of the ¹H-NMR spectrum and had excellent stability.

### 3. Fabrication of Nonaqueous Electrolyte Solution Secondary Battery

LiNi_{1/3}Co_{1/3}Mn_{1/3}O₂ as a positive electrode active material and carbon black as an electrical conductivity-imparting agent were dry-mixed. The obtained mixture was uniformly dispersed in N-methyl-2-pyrrolidone (NMP) in which polyvinylidene fluoride (PVDF) as a binder had been dissolved, thereby preparing a slurry. The obtained slurry was applied to both surfaces of an aluminum metal foil (rectangular, a thickness of 20 µm). The coating film was dried to remove the NMP and the whole film was pressed to obtain a positive electrode sheet having the aluminum metal foil as a positive electrode collector and positive electrode active material layers formed on both surfaces of the foil. A ratio of the solid contents in the positive electrode active material layer of the obtained positive electrode sheet was set to positive electrode active material : electrical conductivity-imparting agent: PVDF=92 : 4 : 4 in terms of a mass ratio.

A commercially available graphite-coated electrode sheet (manufactured by Hosen Co., Ltd., trade name: Electrode Sheet Negative Electrode Monolayer) was used as a negative electrode sheet.

The negative electrode sheet, a separator made of polyethylene, the positive electrode sheet, the separator made of polyethylene, and the negative electrode sheet were laminated in this order in each of the nonaqueous electrolyte solutions obtained in Examples and Comparative Examples to manufacture a battery element. This battery element was put into a bag formed of a laminated film having aluminum (thickness: 40 µm) and resin layers coating both sides thereof such that the terminals of the positive electrode sheet and the negative electrode sheet protruded from the bag. Subsequently, each of the nonaqueous electrolyte solutions obtained in Examples and Comparative Examples was poured into the bag. The bag was vacuum-sealed, and was sandwiched between glass plates and pressurized in order to increase the adhesiveness between the electrodes, thereby fabricating a sheet-shaped nonaqueous electrolyte solution secondary battery.

### 4. Evaluation of Nonaqueous Electrolyte Solution Secondary Battery

### Discharge Capacity Retention and Internal Resistance Ratio

The obtained nonaqueous electrolyte solution secondary battery was subjected to a charging and discharging cycle test at a charging rate of 0.3 C, a discharging rate of 0.3 C, a charge termination voltage of 4.2 V, and a discharge termination voltage of 2.5 V at 25°C. The discharge capacity retention (%) after 200 cycles and the internal resistance ratio after 200 cycles are shown in Table 3.

Furthermore, the "discharge capacity retention (%)" after 200 cycles is a ratio (percentage) of the discharge capacity (mAh) after 200 cycles in the test with respect to the discharge capacity (mAh) after 10 cycles in the test. The "internal resistance ratio" after 200 cycles is a value expressed in a relative value of the resistance after 200 cycles in the test in a case where the resistance before the cycle test was defined as 1.

**Table 3**

| | Electrolyte | Solvent | Additive | Discharge capacity retention (%) | Internal resistance ratio |
|---|---|---|---|---|---|
| Ex. 1 | LiPF6 1.0 mol/L | EC/DEC (30/70) vol% | Cpd. 5a 0.5 mass% | 82 | 1.52 |
| Ex. 2 | LiPF6 1.0 mol/L | EC/DEC (30/70) vol% | Cpd. 5a 1.0 mass% | 87 | 1.44 |
| Ex. 3 | LiPF6 1.0 mol/L | EC/DEC (30/70) vol% | Cpd. 5b 1.0 mass% | 84 | 1.51 |
| Ex. 4 | LiPF6 1.0 mol/L | EC/DEC (30/70) vol% | Cpd. 5c 1.0 mass% | 90 | 1.48 |
| Ex. 5 | LiPF6 1.0 mol/L | EC/DEC (30/70) vol% | Cpd. 5d 1.0 mass% | 88 | 1.52 |
| Ex. 6 | LiPF6 1.0 mol/L | EC/DEC (30/70) vol% | Cpd. 6a 1.0 mass% | 85 | 1.56 |
| Ex. 7 | LiPF6 1.0 mol/L | EC/DEC (30/70) vol% | Cpd. 6b 1.0 mass% | 88 | 1.49 |
| Ex. 8 | LiPF6 1.0 mol/L | EC/DEC (30/70) vol% | Cpd. 6c 1.0 mass% | 86 | 1.48 |
| Comp. Ex. 1 | LiPF6 1.0 mol/L | EC/DEC (30/70) vol% | None | 68 | 1.83 |
| Comp. Ex. 2 | LiPF6 1.0 mol/L | EC/DEC (30/70) vol% | PS 1.0 mass% | 71 | 1.68 |
| Comp. Ex. 3 | LiPF6 1.0 mol/L | EC/DEC (30/70) vol% | VC 1.0 mass% | 78 | 1.59 |
| Comp. Ex. 4 | LiPF6 1.0 mol/L | EC/DEC (30/70) vol% | VC 2.0 mass% | 77 | 1.67 |
| Comp. Ex. 5 | LiPF6 1.0 mol/L | EG/DEC (30/70) vol% | FEC 1.0 mass% | 84 | 1.66 |
| Comp. Ex. 6 | LiPF6 1.0 mol/L | EC/DEC (30/70) vol% | FEC 2.0 mass% | 85 | 1.67 |
| Comp. Ex. 7 | LiPF6 1.0 mol/L | EC/DEC (30/70) vol% | Sulfolane 1.0 mass% | 76 | 1.77 |
| Comp. Ex. 8 | LiPF6 1.0 mol/L | EC/DEC (30/70) vol% | Cpd. A 1.0 mass% | 87 | 1.46 |
| Comp. Ex. 9 | LiPF6 1.0 mol/L | EC/DEC (30/70) vol% | Cpd. B 1.0 mass% | 84 | 1.49 |

### Measurement of Amount of Generated Gas

Separately from the batteries used in the cycle test, a nonaqueous electrolyte solution secondary battery having the same configuration including each of the electrolyte solutions of Examples and Comparative Examples was prepared. The battery was subjected to three cycles of an operation in which the battery was charged to 4.2 V at a current corresponding to 0.2 C, and then discharged to 3 V at a current corresponding to 0.2 C at 25°C, thereby stabilizing the battery. Subsequently, the battery was charged again to 4.2 V at a charging rate of 0.3 C and then stored at a high temperature of 60°C for 168 hours. Thereafter, the battery was cooled to room temperature, the volume of the battery was measured by the Archimedes' method, and the amount of generated gas was determined from a change in the volume before and after the storage. The results are shown in Table 4.

**Table 4**

| | Electrolyte | Solvent | Additive | Amountof generated gas (cm3) |
|---|---|---|---|---|
| Ex. 2 | LiPF6 1.0 mol/L | EC/DEC (30/70) vol% | Cpd. 5a 1.0 mass% | 0.32 |
| Ex. 3 | LiPF6 1.0 mol/L | EC/DEC (30/70) vol% | Cpd. 5b 1.0 mass% | 0.39 |
| Ex. 4 | LiPF6 1.0 mol/L | EC/DEC (30/70) vol% | Cpd. 5c 1.0 mass% | 0.41 |
| Ex. 5 | LiPF6 1.0 mol/L | EC/DEC (30/70) vol% | Cpd. 5d 1.0 mass% | 0.36 |
| Ex. 6 | LiPF6 1.0 mol/L | EC/DEC (30/70) vol% | Cpd. 6a 1.0 mass% | 0.39 |
| Ex. 7 | LiPF6 1.0 mol/L | EC/DEC (30/70) vol% | Cpd. 6b 1.0 mass% | 0.41 |
| Ex. 8 | LiPF6 1.0 mol/L | EC/DEC (30/70) vol% | Cpd. 6c 1.0 mass% | 0.37 |
| Comp. Ex. 1 | LiPF6 1.0 mol/L | EC/DEC (30/70) vol% | None | 0.54 |
| Comp. Ex. 3 | LiPF6 1.0 mol/L | EC/DEC (30/70) vol% | VC 1.0 mass% | 0.58 |
| Comp. Ex. 5 | LiPF6 1.0 mol/L | EC/DEC (30/70) vol% | FEC 1.0 mass% | 0.59 |
| Comp. Ex. 8 | LiPF6 1.0 mol/L | EC/DEC (30/70) vol% | Cpd. A 1.0 mass% | 0.37 |
| Comp. Ex. 9 | LiPF6 1.0 mol/L | EC/DEC (30/70) vol% | Cpd. B 1.0 mass% | 0.38 |

### Recovered Capacity after Storage

Separately from the batteries used in the cycle test and the gas generation test, a nonaqueous electrolyte solution secondary battery having the same configuration, including each of the electrolyte solutions of Examples and Comparative Examples, was prepared. The battery was charged to 4.2 V The charged battery was stored at 75°C for 72 hours and then charged to 4.2 V again. Three cycles of this operation were performed over a total of 216 hours. Thereafter, the discharge capacity of the nonaqueous electrolyte solution secondary battery was measured and the value was defined as a recovered capacity after storage. The results are shown in Table 5.

**Table 5**

| | Electrolyte | Solvent | Additive | Recovered capacity after storage (mAh) |
|---|---|---|---|---|
| Ex. 2 | LiPF6 1.0 mol/L | EC/DEC (30/70) vol% | Cpd. 5a 1.0 mass% | 304 |
| Ex. 3 | LiPF6 1.0 mol/L | EC/DEC (30/70) vol% | Cpd. 5b 1.0 mass% | 299 |
| Ex. 4 | LiPF6 1.0 mol/L | EC/DEC (30/70) vol% | Cpd. 5c 1.0 mass% | 302 |
| Ex. 5 | LiPF6 1.0 mol/L | EC/DEC (30/70) vol% | Cpd. 5d 1.0 mass% | 305 |
| Ex. 6 | LiPF6 1.0 mol/L | EC/DEC (30/70) vol% | Cpd. 6a 1.0 mass% | 302 |
| Ex. 7 | LiPF6 1.0 mol/L | EC/DEC (30/70) vol% | Cpd. 6b 1.0 mass% | 298 |
| Ex. 8 | LiPF6 1.0 mol/L | EC/DEC (30/70) vol% | Cpd. 6c 1.0 mass% | 300 |
| Comp. Ex. 1 | LiPF6 1.0 mol/L | EC/DEC (30/70) vol% | None 1.0 mass% | 279 |
| Comp. Ex. 2 | LiPF6 1.0 mol/L | EC/DEC (30/70) vol% | PS 1.0 mass% | 278 |
| Comp. Ex. 3 | LiPF6 1.0 mol/L | EC/DEC (30/70) vol% | VC 1.0 mass% | 284 |
| Comp. Ex. 8 | LiPF6 1.0 mol/L | EC/DEC (30/70) vol% | Cpd. A 1.0 mass% | 292 |
| Comp. Ex. 9 | LiPF6 1.0 mol/L | EC/DEC (30/70) vol% | Cpd. B 1.0 mass% | 286 |

### Reference Signs List

1: nonaqueous electrolyte solution secondary battery, 2:· positive electrode collector, 3: positive electrode active material layer, 4: positive electrode plate, 5: negative electrode collector, 6: negative electrode active material layer, 7: negative electrode plate, 8: nonaqueous electrolyte solution, 9: separator

## Claims

1. Use of an additive comprising a compound represented by Formula (1) in nonaqueous electrolyte solutions of a lithium ion battery, 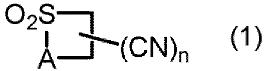 wherein in Formula (1), A represents a divalent hydrocarbon group having 1 to 3 carbon atoms, which may be substituted with a halogen atom, and n represents an integer of 1 to 5.

2. Use according to claim 1,
wherein the compound represented by Formula (1) is a compound represented by Formula (5), 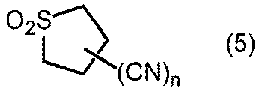
wherein in Formula (5), n represents an integer of 1 to 4.

3. Use according to claim 2,
wherein the compound represented by Formula (1) is a compound represented by Formula (5a):

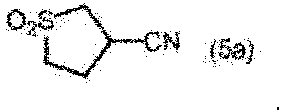

4. A lithium ion battery comprising:
• a nonaqueous electrolyte solution comprising:
(a) the additive for nonaqueous electrolyte solutions as defined in any one of claims 1 to 3;
(b) a nonaqueous solvent; and
(c) an electrolyte
• a positive electrode; and
• a negative electrode.

5. The lithium ion battery according to claim 4, wherein the nonaqueous solvent includes cyclic carbonate and chained carbonate.

6. The lithium ion battery according to claim 4 or 5, wherein the electrolyte includes a lithium salt.

## Patentansprüche

1. Verwendung eines Additivs, das eine durch die Formel (1) dargestellte Verbindung umfasst, in nichtwässrigen Elektrolytlösungen einer Lithium-Ionen-Batterie, 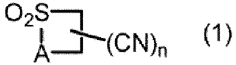 wobei in der Formel (1) A eine zweiwertige Kohlenwasserstoffgruppe mit 1 bis 3 Kohlenstoffatomen darstellt, die mit einem Halogenatom substituiert sein kann, und n eine ganze Zahl von 1 bis 5 darstellt.

2. Verwendung nach Anspruch 1,
wobei die durch die Formel (1) dargestellte Verbindung eine durch die Formel (5) dargestellte Verbindung ist, 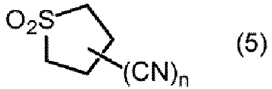
wobei in der Formel (5) n eine ganze Zahl von 1 bis 4 darstellt.

3. Verwendung nach Anspruch 2,
wobei die durch die Formel (1) dargestellte Verbindung eine durch die Formel (5a) dargestellte Verbindung ist:

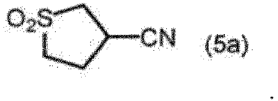

4. Lithium-Ionen-Batterie, die Folgendes umfasst:
• eine nichtwässrige Elektrolytlösung, die Folgendes umfasst:
(a) das Additiv für nichtwässrige Elektrolytlösungen nach einem der Ansprüche 1 bis 3;
(b) ein nichtwässriges Lösungsmittel; und
(c) einen Elektrolyten
• eine positive Elektrode; und
• eine negative Elektrode.

5. Lithium-Ionen-Batterie nach Anspruch 4, wobei das nichtwässrige Lösungsmittel zyklisches Carbonat und Kettencarbonat enthält.

6. Lithium-Ionen-Batterie nach Anspruch 4 oder 5, wobei der Elektrolyt ein Lithiumsalz enthält.

## Revendications

1. Utilisation d'un additif comprenant un composé représenté par la Formule (1) dans des solutions électrolytiques non aqueuses d'une batterie lithium-ion, 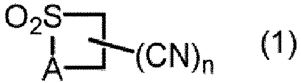 où dans la Formule (1), A représente un groupe hydrocarbone divalent ayant de 1 à 3 atomes de carbone, qui peut être substitué par un atome d'halogène, et n représente un nombre entier de 1 à 5.

2. Utilisation selon la revendication 1,
où le composé représenté par la Formule (1) est un composé représenté par la Formule (5), 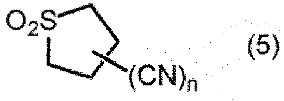
où dans la Formule (5), n représente un nombre entier de 1 à 4.

3. Utilisation selon la revendication 2,
où le composé représenté par la Formule (1) est un composé représenté par la Formule (5a) :

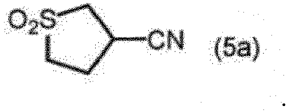

4. Batterie lithium-ion comprenant :
• une solution électrolytique non aqueuse comprenant :
(a) l'additif pour des solutions électrolytiques non aqueuses tel que défini dans l'une quelconque des revendications 1 à 3 ;
(b) un solvant non aqueux ; et
(c) un électrolyte
• une électrode positive ; et
• une électrode négative.

5. Batterie lithium-ion selon la revendication 4, où le solvant non aqueux inclut du carbonate cyclique et du carbonate en chaîne.

6. Batterie lithium-ion selon la revendication 4 ou 5, où l'électrolyte inclut un sel de lithium.
